# EUROPEAN PATENT SPECIFICATION

(11) **EP 0 918 521 B1**
(45) Date of publication and mention of the grant of the patent: **25.09.2002**
(21) Application number: 97934492.6
(22) Date of filing: 14.07.1997
(51) Int. Cl.: A61K 31/435, A61P 25/28

(54) **FORMULATION FOR THE TREATMENT AND/OR PROPHYLAXIS OF DEMENTIA**
FORMULIERUNG ZUR BEHANDLUNG UND/ODER VORBEUGUNG VON DEMENTIA
FORMULATION POUR LE TRAITEMENT ET/OU LA PROPHYLAXIE DE LA DEMENCE

(30) Priority: 25.07.1996 GB 9615628
(43) Date of publication of application: 02.06.1999
(73) Proprietor: SmithKline Beecham plc, Brentford, Middlesex TW8 9GS (GB)
(72) Inventor: VAN SCHIE, Dirk M. J., SmithKline Beecham Pharmac., Harlow, Essex CM19 5AW (GB)
(74) Representative: Valentine, Jill Barbara
(86) International application number: EP9703872
(87) International publication number: WO98004258

(56) References cited:
- EP-A- 0 392 803
- WO-A-93/17018
- WO-A-95/31456
- WO-A-96/21434

## Description

This invention relates to pharmaceutical compositions for the treatment by transdermal administration.

The skin is the largest and most accessible of body tissues. Hence transdermal delivery of medicinal agents would be a most useful alternative to, and can offer some advantages over, oral delivery. A transdermal formulation offers the advantage of a more convenient mode of administration of the drug substance, thereby potentially enhancing patient compliance. In addition, drug substance is released in a more controlled fashion, over a prolonged period, offering potential therapeutic advantges. However, the skin is highly impermeable to the ingress of most materials and few drugs can be delivered by this route to induce a systemic effect.

[R-(Z)]-α-(methoxyiminoy-α-(1-azabicyclo [2.2.2]oct-3-yl)acetonitrile monohydrochloride (compound X) and methods for its preparation are disclosed in EP-A-0392803, WO95/31456 and WO93/17018. The compound enhances acetylcholine function via an action at muscarinic receptors within the central nervous system, and is therefore of potential use in the treatment and/or prophylaxis of dementia in mammals.

WO96/12486 discloses the use of compound X in the manufacture of a medicament for enhancing amyloid precursor protein processing along a non-amyloidogenic pathway in patients suffering from, or at risk of developing, Alzheimer's disease.

We have now found that compound X can be delivered through human skin at levels well above those required therapeutically.

Accordingly, the present invention provides the use of compound X in the manufacture of a medicament for the treatment and/or prophylaxis of dementia in humans, wherein the medicament is adapted for application to the skin.

The present invention also provides a compositions for treatment and/or prophylaxis of dementia in humans by adminstration of compound X to the skin.

The compound will be administered as a suitable pharmaceutical composition for administration to the skin, for example as an ointment or gel, or as a transdermal patch, either as containing a formulation such as a gel, or as a transdermal patch in the form of a matrix formulation.

It will be appreciated that all references herein to compound X include the free base form and also pharmaceutically acceptable salts, such as the hydrochloride.

A pharmaceutical composition for the method of treatment of the present invention may be administered preferably by continuous administration using a transdermal delivery device.

The present invention also provides a pharmaceutical composition for administration to the skin, which comprises compound X together with a suitable pharmaceutically acceptable carrier.

The compound will preferably be presented in a liquid or semi-solid formulation which enables an accurate dose of drugs to be applied. For convenience and accuracy of dose the formulation may be contained in a patch, to enable a precise area of the skin to be exposed to the formulation, and which forms an occlusive cover which may facilitate skin hydration and enhance delivery. Alternative formulations are also possible, for instance a metered dose spray or a tube delivering an accurate dose of ointment or gel.

Ointments, gels and sprays, are formulated as known in the art, for example as described in standard text books of pharmaceutics and cosmetics, such as Harry's Cosmeticology published by Leonard Hill Books.

Gels - Gels are semisolid systems consisting either of suspensions made up of small inorganic particles or of large organic molecules interpenetrated by a liquid. Where the gel consists of a network of small discrete particles, the gel is classified as a two-phase system. In a two-phase gel, if the particle size of the dispersed phase is relatively large, the gel is sometimes referred to as a magma. Both gels and magmas may be thixotropic, forming semisolids on standing and becoming liquid on agitation. They should be shaken before use to ensure homogeneity.

Single-phase gels consist of organic macromolecules uniformly distributed throughout a liquid so that no apparent boundaries exist between the dispersed macromolecules and the liquid. Single-phase gels may be made from synthetic macromolecules (e.g. Carbomer) or from natural gums (e.g. Tragacanth). The latterpreparations are also called mucilages. Although single-phase gels are commonly aqueous, alcohols and oils may also be used as the continuous phase. For example, mineral oil can be combined with a polyethylene resin to form a gel which may be used as an oleaginous ointment base. (Formulations that are capitalized in this discussion are classified as pharmacopeial preparations (USPharmacopeia).)

Spray - Spray formulations are aqueous solutions of various drugs which are applied to the skin from a container having a spray means (e.g., an atomizer or nebulizer).

The present invention further provides a method for the preparation of a pharmaceutical composition for administration to the skin, as hereinbefore defined, which comprises mixing compound X with a suitable pharmaceutically acceptable carrier.

The compositions may also contain anti-oxidants and other conventional ingredients such as preservatives, perfumes, alcohol and, advantageously, a penetrating agent such as alkylmethylsulphoxides, [including DMSO (dimethylsulphoxide) and decylmethylsulphoxide], n-methylpyrrolidone, isopropyl myristate or propan-2-ol.

The compound may be administered by means of a transdermal delivery system, for example, as described in Drugs and the Pharmaceutical Sciences Volume 32, Tyle P. 'Drug delivery devices - fundamentals and applications'; and Journal of Controlled Release, 4 (1987) 237-251 Guy R. Transdermal Drug Delivery: A perspective'. Such devices have the advantage over other means of delivery in that an accurate dose can be applied and the delivery is continuous and multiple applications are not necessary.

Suitable transdermal formulations are well known in the art (see for instance Percutaneous Absorption and Transdermal Therapy, K A Walters, March 1986; Pharmaceutical Dosage Forms and Drug Delivery Systems, (5th Ed.), H C. Ansel and N G. Popovich, Chapter 9, Lea and Febiger (1990), pages 307 to 320 and Sustained and Controlled Release Drug Delivery Systems, ed J R Robinson, Marcel Dekker Inc., New York (1978), pages 579 *et seq*.). Two main types of transdermal delivery devices are currently marketed and these are classified as matrix and membrane systems (Physicochemical Principles of Pharmacy, A.T. Florence and D. Attwood, 2nd Edition, Macmillan, 1993, page 331). In matrix systems, the drug is dispersed in a release controlling matrix which consists either of a gel or an adhesive film. Membrane systems generally consist of a drug reservoir, a rate-controlling membrane and an adhesive layer. In both cases the active is dissolved or suspended in a vehicle which then forms an integral part of the delivery device. The drug substance may be dissolved or suspended in a liquid or a gel. Suitable vehicles include both aqueous and non-aqueous vehicles, for instance saline and saline/propylene glycol (1:1). A penetration enhancer may also be added, if appropriate.

Suitably, the transdermal formulation is provided in the form of a medicated plaster or patch, preferably a patch. Suitably the patch is between 10 and 50cm², preferably between 20 and 40 cm². The patch will be provided with a pharmaceutically accetable adhesive layer so that it can retained on the skin of the user. Preferably the adhesive effect of the layer will be reversible such that the patch will remain in place for the lifetime of the patch but still be easy for the patient or carer to apply and remove.

Methods for the manufacture of transdermal devices are conventional as described in, for example, Hans P. Merkle "Transdermal Delivery Systems" (Meth and Find Exp Clin Pharmacol 1989; 11(3); 135-153) (and references therein).

Preferably, the delivery profile will provide a steady rate delivery. Alternatively, a compartmentalised rate controlled device may used. A suitable target skin flux will be in the range 0.01 to 1, preferably 0.1 to 0.5 µg/cm²/hr.

Suitably, the amount of compound X administered through a transdermal formulation according to the present invention will be selected so that it will provide an amount of drug substantially similar to that obtained following conventional oral administration of a tablet formulation, that is substantially similar to that obtained following administration of 5 to 75µg compound X twice a day, assuming about 50% bioavailablity.

A typical formulation suitable for treating an adult human will suitably contain 1 to 100ppm, preferably 10 to 50ppm of compound X.

Suitably the transdermal formulation is provided in unit dose form. Suitably, the transdermal formulation is provided in a range of dosage amounts, for instance to allow for titration of an individual patient's drug requirement. A suitable dose may be obtained by combining different strength formulations. Suitably, the unit dose form will provide sufficient drug substance for a period of 1-7 days (including, if appropriate 'off time'), to permit once-a-day to once-a week application of the formulation. Suitably, the transdermal formulation will be administered for a period of continuous therapy.

### Example 1: in- vitro Percutaneous Penetration of Compound X

The potential of compound X for formulation into a transdermal delivery system was initially evaluated by determining drug penetration from a solution using a human epidermis *in vitro* model.

The *in vitro* percutaneous penetration method utilised concentrated (100mg/mL) solutions of Compound X in 70% aqueous isopropanol. The *in vitro* set up consisted of modified Franz cells (Dermatological Formulations: Percutaneous Absorption. B.W. Barry, Marcel Dekker, 1983, 245) with human epidermal membrane and 12mL of a receptor fluid consisting of 70% aqueous isopropanol. Eleven cells were used, with a surface area of epidermis of 0.79 cm² and 200µL of donor solution applied to each. Samples were taken from the receptor after 24 and 45 hours and analysed for compound X content. From these results the concentration of compound X penetrating human epidermis with time could be determined.

### Results

Over the first 24 hours, the average amount of compound X which permeated the epidermis was 1800 µg.cm⁻²; over the entire 45 hours, the average amount which permeated was 15000 µg.cm⁻²

### Conclusions

This experiment shows clearly that it is feasible to deliver compound X through human skin at levels well above those required therapeutically

### Example 2

A typical patch comprising a membrane is as follows:
a backing layer of aluminized plastic that is impermeable to compound X;
drug reservoir containing compound X (1 to 100ppm) in a saline/propylene glycol (1:1) vehicle;
ethylene-vinyl acetate copolymer membrane that is permeable to compound X; and
a layer of hypoallergenic silicone adhesive;
plus a protective peel strip covering the adhesive surface.
patch size = 20-40cm²
reservoir volume = 0.5-1ml

### Example 3

A typical patch comprising a matrix is as follows:
backing foil;
drug reservoir comprising a compound X/lactose trituration homogeneously dispersed in a hydrogel composed of water, glycerin, poly vinyl alcohol and polyvinylpyrrolidone;
adhesive layer; and
a release liner.

## Claims

1. The use of [R-(Z)]-α-(methoxyimino)-α-(1-azabicyclo [2.2.2]oct-3-yl)acetonitrile or a pharmaceutically acceptable salt thereof in the manufacture of a medicament for the treatment and/or prophylaxis of dementia in humans, wherein the medicament is adapted for application to the skin.

2. A pharmaceutical composition for administration to the skin, which comprises [R-(Z)]-α-(methoxyimino)-α-(1-azabicyclo [2.2.2]oct-3-yl)acetonitrile or a pharmaceutically acceptable salt thereof together with a suitable pharmaceutically acceptable carrier.

3. A use according to claim 1 or composition according to claim 2 wherein the compound is [R-(Z)]-α-(methoxyimino)-α-(1-azabicyclo[2.2.2]oct-3-yl)acetonitrile monohydrochloride.

4. A use or composition according to any preceding claim wherein the compound is administered using a transdermal delivery device.

5. A use or composition according to claim 4 wherein the device is a matrix system.

6. A use or composition according to claim 4 wherein the device is a membrane system.

7. A use or composition according to claim 4, 5 or 6 wherein the device is a patch.

8. A use or composition according to claim 4, 5, 6 or 7 wherein the device has a delivery surface area between 10 and 50cm².

9. A use or composition according to claim 7 or 8 wherein the device provides a skin flux in the range 0.01 to 1µg/cm²/hr.

10. A use or composition according to any preceding claim which provide an amount of compound substantially similar to that obtained following oral administration of 5 to 75µg compound twice a day, assuming about 50% bioavailablity.

11. A use or composition according to any preceding claim wherein the compound is administered in unit dose form containing 1 to 100ppm of compound.

## Patentansprüche

1. Verwendung von [R-(Z)]-α-(Methoxyimino)-α-(1-azabicyclo[2.2.2]oct-3-yl)acetonitril oder eines pharmazeutisch verträglichen Salzes davon bei der Herstellung eines Medikamentes für die Behandlung von und/oder die Prophylaxe von Demenz beim Menschen, wobei das Medikament für die Anwendung auf der Haut angepaßt ist.

2. Pharmazeutische Zubereitung für die Anwendung auf der Haut, die [R-(Z)]-α-(Methoxyimino)-α-(1-azabicyclo[2.2.2]oct-3-yl)acetonitril oder ein pharmazeutisch verträgliches Salz davon zusammen mit einem geeigneten pharmazeutisch verträglichen Träger umfasst.

3. Verwendung nach Anspruch 1 oder Zubereitung nach Anspruch 2, wobei die Verbindung [R-(Z)]-α-(Methoxyimino)-α-(1-azabicyclo[2.2.2]oct-3-yl)acetonitrilmonohydrochlorid ist.

4. Verwendung oder Zubereitung nach einem der vorstehenden Ansprüche, wobei die Verbindung mit einer Einrichtung für die transdermale Abgabe verabreicht wird.

5. Verwendung oder Zubereitung nach Anspruch 4, wobei die Einrichtung ein Matrixsystem ist.

6. Verwendung oder Zubereitung nach Anspruch 4, wobei die Einrichtung ein Membransystem ist.

7. Verwendung oder Zubereitung nach Anspruch 4, 5 oder 6, wobei die Einrichtung ein Pflaster ist.

8. Verwendung oder Zubereitung nach Anspruch 4, 5, 6 oder 7, wobei die Einrichtung eine Oberfläche für die Abgabe von 10 bis 50 cm² hat.

9. Verwendung oder Zubereitung nach Anspruch 7 oder 8, wobei die Einrichtung einen Hautfluss im Bereich von 0,01 bis 1 µg/ cm²/h bereitstellt.

10. Verwendung oder Zubereitung nach einem der vorstehenden Ansprüche, die eine Menge der Verbindung bereitstellt, die der im Wesentlichen ähnlich der ist, die nach einer täglich zweimaligen oralen Verabreichung von 5 bis 75 µg der Verbindung erhalten wird, wobei eine biologische Verfügbarkeit von etwa 50 % angenommen wird.

11. Verwendung oder Zubereitung nach einem der vorstehenden Ansprüche, wobei die Verbindung in der Form einer Dosierungseinheit verabreicht wird, die 1 bis 100 ppm der Verbindung enthält

## Revendications

1. Utilisation de [R-(Z)]-α-(méthoximino)-α-(1-azabicyclo[2.2.2]oct-3-yl)acétonitrile ou d'un de ses sels pharmaceutiquement acceptables dans la production d'un médicament destiné au traitement et/ou à la prophylaxie de la démence chez l'homme, dans laquelle le médicament est adapté à l'application à la peau.

2. Composition pharmaceutique pour l'administra-tion à la peau, qui comprend du [R-(Z)]-α-(méthoximino)-α-(1-azabicyclo[2.2.2]oct-3-yl)acétonitrile ou d'un de ses sels pharmaceutiquement acceptables conjointement avec un support convenable pharmaceutiquement acceptable.

3. Utilisation suivant la revendication 1 ou composition suivant la revendication 2, dans laquelle le composé est le monochlorhydrate de [R-(Z)]-α-(méthoximino)-α-(1-azabicyclo[2.2.2]oct-3-yl)acétonitrile.

4. Utilisation ou composition suivant l'une quelconque des revendications précédentes, dans laquelle le composé est administré en utilisant un dispositif d'administration transdermique.

5. Utilisation ou composition suivant la revendication 4, dans laquelle le dispositif est un système à matrice.

6. Utilisation ou composition suivant la revendication 4, dans laquelle le dispositif est un système à membrane.

7. Utilisation ou composition suivant la revendication 4, 5 ou 6, dans laquelle le dispositif est un timbre.

8. Utilisation ou composition suivant la revendication 4, 5, 6 ou 7, dans laquelle le dispositif a une surface d'administration de 10 à 50 cm².

9. Utilisation ou composition suivant la revendication 7 ou 8, dans laquelle le dispositif provoque un flux cutané compris dans l'intervalle de 0,01 à 1 µg/cm²/h.

10. Utilisation ou composition suivant l'une quelconque des revendications précédentes, qui fournit une quantité de composés pratiquement similaire à celle obtenue après administration orale de 5 à 75 µg de composé deux fois par jour, en considérant une biodisponibilité d'environ 50 %.

11. Utilisation ou composition suivant l'une quelconque des revendications précédentes, dans lesquelles le composé est administré dans une forme posologique unitaire contenant 1 à 100 ppm du composé.
